**Europäisches Patentamt**

**European Patent Office**

**Office européen des brevets**

(11) Numéro de publication: **0 259 212 B1**

## FASCICULE DE BREVET EUROPEEN

(45) Date de publication de fascicule du brevet: **16.02.94**

(51) Int. Cl.⁵: **C12N 15/00**, C12N 7/00, C12N 5/00, A61K 37/02, A61K 35/76, A61K 39/12, C07K 15/00, G01N 33/574

(21) Numéro de dépôt: **87401866.6**

(22) Date de dépôt: **11.08.87**

(54) **Expression d'un antigène spécifique de tumeur par un virus vecteur recombinant et utilisation de celui-ci pour le traitement préventif ou curatif de la tumeur correspondante.**

(30) Priorité: **13.08.86 FR 8611700**

(43) Date de publication de la demande:
**09.03.88 Bulletin 88/10**

(45) Mention de la délivrance du brevet:
**16.02.94 Bulletin 94/07**

(84) Etats contractants désignés:
**AT BE CH DE ES FR GB GR IT LI LU NL SE**

(56) Documents cités:
**EP-A- 0 110 385**
**EP-A- 0 176 170**

**PROCEEDINGS OF THE NATIONAL ACADEMY OF SCIENCES OF THE USA, vol. 82, no. 5, mars 1985, pages 1359-1363, Washington, US; S.L. MANSOUR et al.: "An adenovirus vector system used to express polyoma virus tumor antigens"**

(73) Titulaire: **TRANSGENE S.A.**
**11, rue de Molsheim**
**F-67000 Strasbourg(FR)**

(72) Inventeur: **Lathe, Richard**
**Arc Abro**
**Kings Buildings**
**West Mains Road**
**Edinburgh EH9(GB)**
Inventeur: **Kieny, Marie-Paule**
**11, rue de Gascogne**
**F-67100 Strasbourg(FR)**

(74) Mandataire: **Ahner, Francis et al**
**CABINET REGIMBEAU**
**26, avenue Kléber**
**F-75116 Paris (FR)**

PROCEEDINGS OF THE NATIONAL ACADEMY OF SCIENCES USA, vol. 79, février 1982, pages 1059-1063; S.M. DILWORTH et al.: "Monoclonal antibodies against polyoma virus tumor antigens"

MOLECULAR AND CELLULAR BIOLOGY, vol. 6, no. 5, mai 1986, pages 1545-1551, American Society for Microbiology, New York, US; S. KORNBLUTH et al.: "Transformation of chicken embryo fibroblasts and tumor induction by the middle T antigen of polyomarvirus carried in an avian retroviral vector"

PROCEEDINGS OF THE NATIONAL ACADEMY OF SCIENCES USA, vol. 83, juin 1986, pages 4307-4311; VAN CHERINGTON et al.: "Recombinant retroviruses that transduce individual polyoma tumor antigens: effects on growth and differentiation"

## Description

Les cellules tumorales, qu'elles soient spontanées ou induites par des virus, peuvent présenter de nouveaux antigènes à leur surface (1). Des antigènes spécifiques de tumeurs (antigènes T) ont déjà été utilisés pour le diagnostic (2) et la visualisation (3) de carcinomes humains.

Plus récemment on a envisagé leur utilisation comme cibles pour une thérapie anti-tumorale spécifique. L'administration d'anticorps anti-T, soit libres soit fixés à des toxines ou à des isotopes radioactifs a déjà donné des résultats encourageants dans le traitement de certains cas cliniques (4).

Par ailleurs, on peut aussi tenter de stimuler les réponses immunitaires propres de l'hôte contre les antigènes T, ceci suivant différentes approches comme l'inoculation de cellules exprimant l'antigène T tuées (5-7), l'induction d'anticorps anti-idiotypiques dirigés contre la région variable des immunoglobulines anti-T (8-9), ou l'injection de l'antigène T lui-même (10). Ces expériences sont limitées par la quantité d'antigène disponible et par le fait que la stimulation d'une réponse immunitaire de type cellulaire, particulièrement importante dans l'immunité antitumorale, nécessite la présentation simultanée de l'antigène et des déterminants d'histocompatibilité de l'hôte (11).

La présente invention concerne l'expression d'un antigène T ou d'une partie importante de celui-ci par un virus recombinant vivant, dans le but d'induire, in vivo, une immunité antitumorale. L'antigène T utilisé à titre de modèle expérimental et qui sera développé dans les exemples est celui qui apparaît à la surface des cellules de rats transformées par le virus du polyome (PY) (12-14).

Le virus du polyome induit plusieurs types de tumeurs chez les rongeurs. L'induction des tumeurs implique l'intégration de l'ADN viral dans le génome de l'hôte et l'expression de gènes précoces du virus (15-17). L'inoculation de rongeurs avec des cellules transformées par PY, tuées, permet d'induire une immunité contre une inoculation d'épreuve avec des cellules tumorales induites par PY (12-13). Toutefois la démonstration de la présence d'antigènes de transplantation spécifiques de la tumeur et de leur relation avec les antigènes T dont la synthèse est dirigée par la région précoce du génome du virus PY n'a pas encore été clairement établie. Cette étude est compliquée par le fait que la région précoce du génome du PY code simultanément pour 3 protéines distinctes (17) appelées "large-T : LT", "middle-T : MT" et "small-T : ST", en rapport avec leurs poids moléculaires respectifs ; ces 3 antigènes ont la même séquence N-terminale et sont reconnus par les mêmes anticorps polyclonaux.

Comme cela sera décrit dans les exemples, les 3 antigènes T ont été clonés et exprimés séparément, pour préciser leurs rôles et leurs potentiels respectifs.

L'utilisation du virus de la vaccine comme vecteur de clonage et d'expression d'antigènes étrangers a déjà été décrite (18-19). Des virus recombinants exprimant des antigènes de virus hétérologues ou de parasites on été utilisés pour immuniser des animaux contre le pathogène correspondant (voir revue dans la référence 20). Les antigènes exprimés par le virus de la vaccine recombinant sont correctement présentés à la surface des cellules infectées et permettent l'induction d'une réponse immunitaire de type cellulaire (21-24), ce qui est particulièrement favorable puisqu'il est connu que l'élimination des cellules tumorales implique l'immunité cellulaire (25,26).

La présente invention concerne l'utilisation d'un virus comme vecteur d'expression d'une séquence d'ADN codant pour la région essentielle d'une protéine spécifique d'une tumeur, appelée antigène T.

Par protéine spécifique d'une tumeur on entend un antigène spécifique d'une tumeur spontanée et absent des tissus adultes normaux ou un antigène codé par un virus oncogène, agent causatif des ladite tumeur.

Par région essentielle de ladite protéine on entend désigner la partie de la séquence protéique capable d'induire une immunité antitumorale ou d'induire un mécanisme capable de faire régresser ladite tumeur.

Il faut noter que, bien que la vaccine soit préférée comme virus vecteur, d'autres virus pourraient également être utilisés, en particulier les virus du groupe de l'Herpes et les Adenovirus.

Le virus comportera l'ensemble des éléments nécessaires à l'expression de la protéine T dans les cellules supérieures, en particulier il comportera un promoteur du virus vecteur. Dans le cas de la vaccine, on utilisera par exemple celui de la protéine de 7,5 Kd. L'ensemble promoteur-séquence codante T sera inséré dans un gène non essentiel du virus : par exemple, dans le cas de la vaccine, le gène de la thymidine kinase (TK), ce qui permettra une sélection aisée des virus recombinants TK⁻.

La présente invention concerne également des cellules supérieures infectées par les virus décrits plus haut ainsi qu'un procédé de préparation, à partir de ces cellules, d'un antigène T caractérisé en ce qu'il comporte la région essentielle d'une protéine spécifique de tumeur.

Les conditions de culture des cellules et des virus sont connues de l'homme de métier et varient suivant les cellules et les virus utilisés, de même que les techniques de séparation de la protéine, celle-ci pouvant être utilisée à des degrés de purification plus ou moins poussés.

La présente invention a pour objet des compositions pharmaceutiques préventives ou curatives des tumeurs présentant l'antigène T, contenant à titre d'agent actif soit le virus selon l'invention, défini dans les revendications 1 à 7.

Dans un premier mode de mise en oeuvre de la présente invention, le virus vivant, exprimant l'antigène T, est inoculé à l'homme ou à l'animal.

Dans un second mode de mise en oeuvre de la présente invention, une préparation pharmaceutique comprenant des extraits de cellules infectées par le virus recombinant ou l'antigène T complètement purifié à partir de ces extraits ou encore le virus recombinant lui-même, tué, présentant à sa surface les molécules d'antigène T, est injectée à l'homme ou à l'animal.

Les compositions pharmaceutiques selon la présente invention seront utilisées de préférence sous forme injectable, à l'homme ou à l'animal.

Ces compositions pharmaceutiques peuvent être préparées selon des procédés connus dans le domaine des vaccins et du conditionnement des protéines.

Les doses applicables pourront varier dans une large gamme et seront fonction, notamment, de la tumeur à prévenir ou à traiter, de l'état du patient et d'autres paramètres qui seront évalués par le praticien.

Les exemples suivants illustrent le concept sur lequel repose l'invention, dans un modèle animal constitué de cellules tumorales de rat et d'un antigène T codé par le virus du polyome, PY, responsable de la tumorigénisation mais il est évident que des résultats semblables pourraient être obtenus avec tout autre antigène spécifique de tumeur et absent des tissus adultes normaux, et que l'utilisation de ces différents antigènes T fait également partie de l'invention.

Parmi les autres antigènes T qui peuvent servir de cible au procédé selon la présente invention, on peut citer les antigènes spécifiques du carcinome colorectal, du mélanome, du cancer du rein, du neuroblastome, du carcinome de la vessie, du carcinome mammaire, des lymphomes et des adénomes des glandes endocrines.

Les antigènes tumoraux servant de cible à la présente invention peuvent être des composants de l'hôte dont l'expression est altérée dans le tissu tumoral ou, plus généralement, des antigènes codés par des virus responsables de la transformation des cellules en cellules cancéreuses, en particulier des rétrovirus ou des Papovavirus comme les papillomes et les polyomes.

Exemple 1 Construction de 3 virus recombinants de la vaccine exprimant chacun l'un des antigènes T du virus PY.

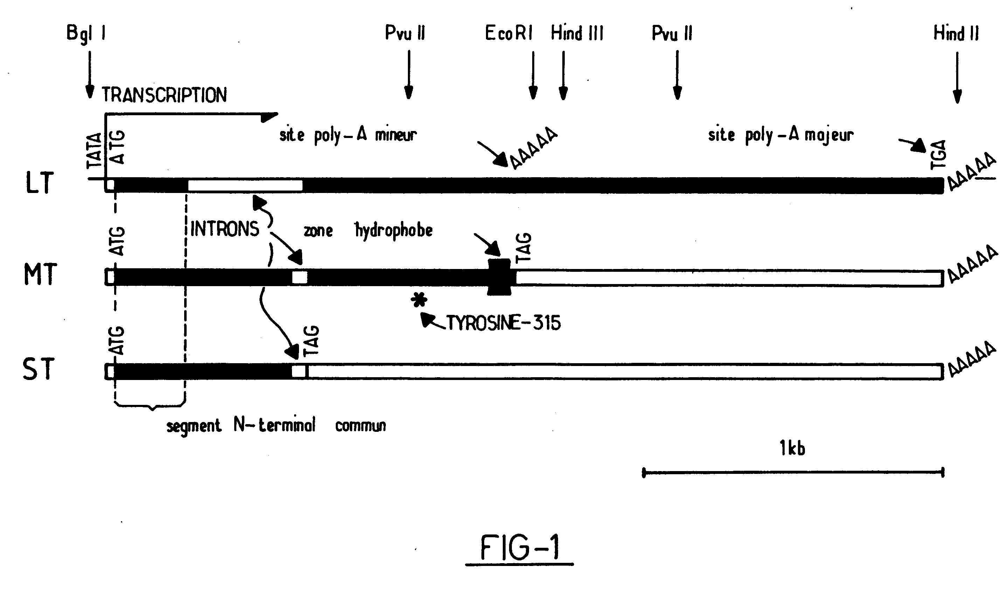

La figure 1 (modifiée d'après la référence 17) montre la structure de la région précoce de l'ADN du PY et met en évidence les 3 gènes LT, MT et ST qui se chevauchent et, en particulier, la partie correspondant à la région N-terminale qui leur est commune. Le site d'initiation de transcription, dans le PY, est indiqué par TATA et les signaux d'initiation et de terminaison de traduction sont notés respectivement ATG et TGA. La position des introns est indiquée ainsi que celle des sites de polyadénylation.

Les séquences codantes des 3 antigènes T du virus PY ont déjà été clonées après avoir été débarrassées de leurs introns par excision in vitro (27-28), pour donner les plasmides pPY-LT1, pPY-MT1, pPY-ST1 (28). Ces séquences codantes ont été récupérées par digestion BglI à une extrémité et HindII pour LT, EcoRI pour MT et PvuII pour ST, à l'autre extrémité. L'extrémité BglI a été rendue compatible avec un site BamHI par un adaptateur synthétique simple brin (42) :

5'-d.GATCTGG-3'

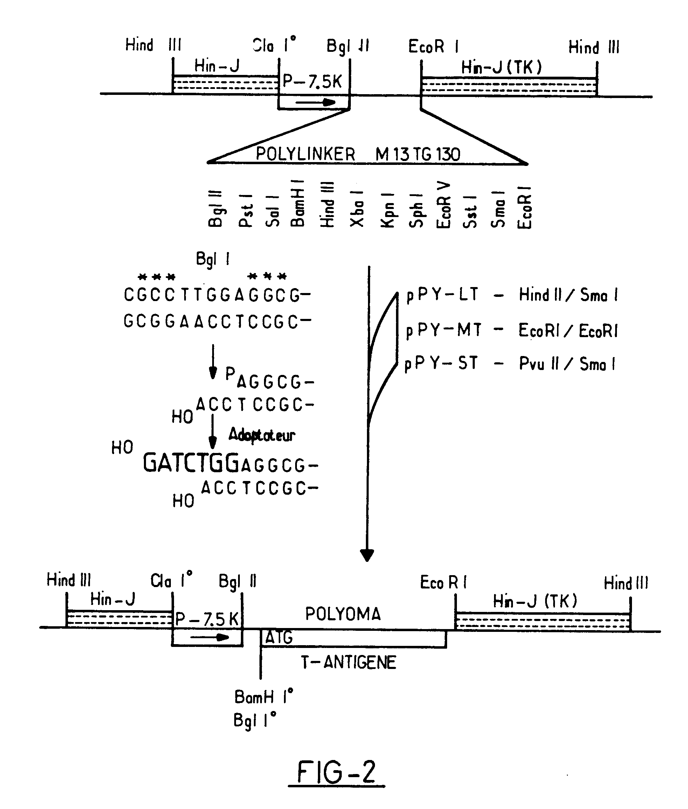

Les 3 segments d'ADN ainsi traités ont été introduits dans le vecteur pTG186-poly respectivement entre les sites BamHI-SmaI, BamHI-EcoRI et BamHI-SmaI (figure 2). Le vecteur pTG186 poly résulte de l'insertion du polylinker de M13TG131 (41) qui apporte plusieurs sites de restriction dans le vecteur pTG1H-TK-7,5 K (29), en aval du promoteur du gène 7,5 K du virus de la vaccine, lui-même inséré dans le gène TK de la vaccine. Les 3 séquences codantes des antigènes T et leurs signaux d'initiation et de terminaison de traduction respectifs ont donc été insérés dans un gène non essentiel du virus de la vaccine, après avoir été placés en aval d'un promoteur du virus de la vaccine. A la suite d'une double recombinaison, dans les cellules infectées par le virus sauvage et transfectées par le plasmide recombinant, comme cela a déjà été décrit précédemment (29), la séquence codante de l'antigène T se retrouve intégrée dans le génome du virus de la vaccine. Les virus recombinants, portant le gène étranger, sont sélectionnés pour leur caractère TK⁻, par multiplication sur cellules 143 B, TK⁻, en présence de 5-bromo-déoxyuridine.

Les virus recombinants ayant intégré les séquences codantes des antigènes T ont été identifiés par "Southern blot" et 3 virus représentatifs ont été retenus : VV.PY.LT-H, VV.PY.MT-I, VV.PY.ST-J.

Exemple 2 Etude in vitro des propriétés des antigènes T synthétisés par les virus recombinantes VV-PY.

Les 3 antigènes T exprimés par les virus de la vaccine recombinants sont reconnus par des anticorps dressés contre l'antigène T du PY natif.

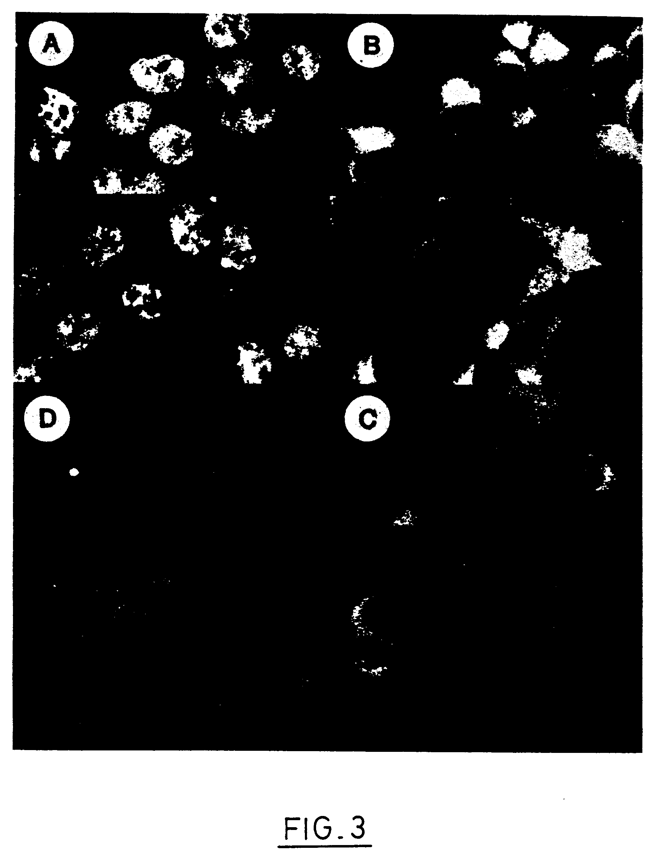

La localisation intracellulaire des antigènes a été étudiée à l'aide d'anticorps fluorescents : des cellules de hamster (BHK21) semi-confluentes ont été infectées séparément avec les 3 types de virus recombinants VV.PY.T (0,1 ufp/cellule ; incubation pendant une nuit) ; les antigènes T ont été révélés, dans les cellules fixées à l'acétone (à 80 %), par application séquentielle d'antiserum de rat hyperimmun, anti-T (RAF n°4, CNRS-Nice (43); 1/50 en PBS + 1 % serumalbumine bovine), puis d'anticorps anti-rat, fluorescents (immunoglobulines de chèvre, fournies par Miles, 1/100 en PBS + 1 % serumalbumine bovine). Les 2 anticorps sont adsorbés chacun pendant 20 minutes à 37°C puis les cellules sont lavées pour éliminer les anticorps non fixés. La figure 3 montre l'immunofluorescence des cellules infectées avec VV.PY.LT (A), VV.PY.MT (B), VV.PY.ST (C) et non infectées (D).

On voit, dans la figure 3 a, que la protéine LT se trouve localisée, comme on l'attend (30, 16), exclusivement dans le noyau ; la protéine ST est localisée principalement dans le cytoplasme (figure 3 c) ; la protéine MT est localisée principalement dans le cytoplasme (figure 3 b) et non à la surface, comme cela a été rapporté (15, 31). Sa détection faible mais reproductible dans la région périnucléaire suggère une association avec l'appareil de Golgi et d'autres membranes intracellulaires, comme cela a été indiqué par d'autres études récentes (32-33).

Exemple 3 Etude de la réponse immunitaire induite chez les animaux inoculés par voie sous-cutanée avec les virus recombinants.

Afin d'évaluer le potentiel vaccinant des 3 recombinants VV-PY, leur capacité d'induire une réponse immunitaire antitumorale a été évaluée in vivo.

On sait que des rats inoculés par voie sous cutanée avec des cellules syngéniques transformées par le PY développent rapidement des tumeurs, localisées au site de la transplantation. Ce modèle expérimental doit donc permettre la mise en évidence de l'induction d'une réponse immunitaire capable de bloquer le développement de la tumeur.

Des groupes de rats femelles (Fischer) âgés de 4 semaines ont été inoculés par voie sous-cutanée avec les différents virus recombinants (à une dose de $10^7$ ufp dans 100 $\mu$l), réinoculés avec la même dose après 12 jours puis soumis à une inoculation d'épreuve, le 16ème jour, avec des cellules de rat 3T3 transformées par le PY complet (PYT-21), à une dose de $2.10^4$ cellules dans 100 $\mu$l. Un groupe de rats a été inoculé avec les 3 virus recombinants simultanément, pour mettre en évidence un éventuel effet cumulatif des 3 antigènes T, comme cela a déjà été suggéré (28). Les résultats sont présentés dans le tableau I :

- Tous les animaux contrôles, non vaccinés, développent des tumeurs détectables dans les 14 jours suivant l'inoculation des cellules transformées ; de plus, on n'observe aucune régression spontanée pendant la durée de l'expérience (42 jours).
- Les animaux vaccinés avec VV.PY.LT et VV.PY.MT développent de petites tumeurs (d'un diamètre ≥ 5 mm) qui régressent rapidement pour être totalement éliminées chez 50 à 60 % des animaux.
- Les animaux vaccinés avec VV.PY.ST développent des tumeurs qui ne régressent pas au cours du temps. L'effet observé avec VV.PY.LT et MT est donc bien spécifique et ne peut pas être attribué à une stimulation non spécifique du système immunitaire par le virus de la vaccine, qui est un très bon immunogène et aurait pu avoir un mode d'action semblable à celui du BCG (34).

- L'inoculation simultanée des 3 recombinants n'apporte pas d'amélioration significative.

Tableau I      Rejet des tumeurs par les rats inoculés par voie sous-cutanée avec les recombinants VV.PY.T

| Virus vaccinal | Nombre d'animaux rejetant la tumeur / Nombre total inoculé | | | |
|---|---|---|---|---|
| | Temps après le challenge : 18 jours | 21 jours | 27 jours | 35 jours |
| - | 0/4 | 0/4 | 0/4 | 0/4 |
| VV.PY.ST | 0/7 | 0/7 | 0/7 | 0/7 |
| VV.PY.MT | 0/10 | 1/10 | 3/10 | 6/10 |
| VV.PY.LT | 0/10 | 0/10 | 2/10 | 5/10 |
| VV.PY.LT +VV.PY.MT +VV.PY.ST | 2/4 | 2/4 | 1/4 | 1/4* |

Note : * 1 animal n'a pas développé de tumeur détectable jusqu'au 35ème jour après le challenge.

Les animaux ont été sacrifiés après 42 jours et leurs sérums ont été analysés (Tableau II). Tous les animaux vaccinés présentent des titres d'anticorps élevés contre le virus de la vaccine et contre les cellules transformées par PY, qu'ils aient été ou non capables de rejeter les tumeurs.

Il semble donc que le rejet des tumeurs ne puisse pas être attribué aux anticorps circulants mais nécessite la participation d'un autre mécanisme immunitaire.

Tableau II    Titres d'anticorps des animaux vaccinés et chal-
              lengés, sacrifiés après 42 jours.

| Virus vaccinal | Diamètre de la tumeur (en mm) | Titre en anticorps* contre | | | |
|---|---|---|---|---|---|
| | | la vaccine | (moyenne) | les cellules trans-formées par PY | (moyenne) |
| – | 15 | 1.8 | | 13.7 | |
| | 20 | 6.9 | (7.9) | 10.2 | (12.3) |
| | 15 | 15.9 | | 7.3 | |
| | 5 | 6.7 | | 18.0 | |
| VV.PY.ST | 15 | 253 | | 56.5 | |
| | 10 | 220 | (224) | 26.1 | (37.0) |
| | 15 | 218 | | 31.6 | |
| | 20 | 203 | | 33.8 | |
| VV.PY.MT | – | 227 | | 35.9 | |
| | – | 258 | (243) | 32.6 | (30.7) |
| | – | 266 | | 28.4 | |
| | 10 | 222 | | 25.9 | |
| VV.PY.LT | – | 226 | | 11.8 | |
| | 14 | 226 | (243) | 21.2 | (37.9) |
| | – | 227 | | 30.9 | |
| | – | 295 | | 87.8 | |
| VV.PY.LT +VV.PY.MT +VV.PY.ST | – | 180 | | 16.0 | |
| | 15 | 300 | (247) | 23.4 | (22.8) |
| | 3 | 252 | | 33.9 | |
| | 8 | 255 | | 17.9 | |

* Les anticorps sont titrés en microplaques avec le virus de
  la vaccine purifié ($10^6$ ufp dans 100 µl) ou une suspension
  de cellules PYT-21 ($10^5$ cellules dans 100 µl) ; l'anticorps

fixé est détecté par addition séquentielle d'IgG de mouton,
anti-rat, marquées à la biotine (Amersham) puis de
streptavidine-peroxydase (Amersham) puis de réactif ELAVIA-
R9 (Pasteur-Diagnostic). L'absorption à 492 nm est mesurée
avec un lecteur de plaques Titertek-Uniskan. Le titre en
anticorps est donné par le produit de la multiplication de
la densité optique mesurée par la dilution de l'antiserum
employée. Chaque résultat est la moyenne de 3 dilutions (1/
50, 1/250 et 1/1.250).

Exemple 4 Rejet des tumeurs chez les animaux vaccinés par voie intradermique avec les virus recombinants.

Une autre voie d'inoculation du virus vaccinal a été essayée.

Des groupes de rats femelles (Fischer) âgés de 4 semaines ont été vaccinés par voie intradermique, par scarification de la base de la queue au scalpel, avec du virus purifié (10 $\mu$l d'un stock à 2.10$^9$ ufp/ml). Une deuxième dose est inoculée après 15 jours. Les animaux sont soumis à l'inoculation d'épreuve, par injection sous-cutanée de 2.10$^4$ cellules PYT-21 dans 100 $\mu$l, 4 jours plus tard.

Les résultats sont présentés dans le tableau III.

### Tableau III  Rejet des tumeurs par les rats inoculés par voie intradermique avec les recombinants VV.PY.T.

| Virus vaccinal | Nombre d'animaux rejetant la tumeur/ Nombre total inoculé | | |
|---|---|---|---|
| | Temps après le challenge : 22 jours | 29 jours | 39 jours |
| | 0/4 | 0/4 | 0/4 |
| VV.PY.ST | 0/4 | 0/4 | 0/4 |
| VV.PY.MT | 2/8 | 7/8 | 8/8 |
| VV.PY.LT | 0/8 | 0/8 | 5/8 |

Exemple 5 Traitement des animaux porteurs de tumeur avec les virus recombinants VV.PY.T.

L'administration des virus recombinants a été tentée comme traitement curatif des tumeurs.

10 rats inoculés avec des cellules transformées par PY et ayant développé une tumeur ont été inoculés avec le recombinant VV.PY.LT après 12 et 16 jours ; la tumeur mesurait 2 à 3 mm au moment de la première inoculation.

Chez tous les animaux la tumeur a continué à croître jusqu'à atteindre un diamètre de 15 mm. Chez 2 animaux la tumeur a ensuite régressé de manière significative (observation au jour 35) puis a été totalement éliminée.

Un résultat semblable n'a été observé ni avec VV.PY.MT ni avec VV.PY.ST ce qui indique des différences d'immunogénicité entre les 3 espèces d'antigène T.

Dépôt de souche représentative de l'invention.

Le plasmide vecteur dans lequel toute séquence codante d'un antigène T peut être intégrée pour être ensuite recombinée dans le virus de la vaccine a été déposé à la Collection Nationale de Cultures des Microorganismes sous le n°I 458, le 20 juin 1985.

Ce plasmide de E. coli dérivé du pML2 comprend une origine de réplication dans E. coli, le gène de la $\beta$-lactamase, le gène TK de la vaccine, interrompu par le promoteur 7,5 K de la vaccine et une séquence codante étrangère (séquence de l'IL2 humaine), qui peut être échangée avec la séquence codante d'un antigène T.

8

REFERENCES

1. Hellstrom, K.E., Hellstrom, I. & Brown, J.P. Springer Semin. Immunopathol. 5, 127-146 (1982).
2. Herlyn, M., Blaszczyk, M. & Koprowski, H. Contr. Oncol. 19, 160-170 (1984).
3. Begent, R.H.J. Biochim. Biophys. Acta 780, 151-166 (1985).
4. Miller, R.A., Maloney, D., Warnke, R., McDougall, R., Wood, G., Kawakami, T., Dilley, J., Goris, M.I. & Levi, R. In: Hybridomas in Cancer Diagnosis and Treatment, Mitchell, M.S. & Oettgen, H.F. (eds.), Raven Press, New York (1982).
5. Gross, L. Cancer Res. 3, 326-333 (1943).
6. Foley, E.J. Cancer Res. 13, 835-837 (1953).
7. Prehn, R.T. & Main, J.M. J. Natl. Cancer Inst. 18, 769-778 (1957).
8. Lee, V.K., Harriott, T.G., Kuchroo, V.K., Halliday, W.J., Hellstrom, I. & Hellstrom, K.E. Proc. Natl. Acad. Sci. USA 82, 6286-6290 (1985).
9. Herlyn, D., Ross, A.H. & Koprowski, H. Science 232, 100-102 (1986).
10. Teventhia, S.S., Flyer, D.C. & Tijan, R. Virology 107,13-23 (1980).
11. Zinkernagel, R.M. & Doherty, P.C. Adv. Immunol. 27, 51-177 (1979).
12. Sjogren, H.O., Hellstrom, I. & Klein, I. Cancer Res. 21, 329-337 (1961).
13. Habel, K. Proc. Soc. Exp. Biol. Med. 106, 722-725 (1961).
14. Ito, Y., Brocklehurst, J.R. & Dulbecco, R. Proc. Natl. Acad. Sci. USA 74, 4666-4670 (1977).
15. Basilico, C. Pharmac. Ther. 26, 235-272 (1984).
16. Griffin, B.E. & Dilworth, S.M. Adv. Cancer Res. 39, 183-268 (1983).
17. Tooze, J. DNA Tumor Viruses, Cold Spring Harbor Press, New York (1981).
18. Panicali, D. & Paoletti, E. Proc. Natl. Acad. Sci. USA 79, 4927-4931 (1982).
19. Mackett, M., Smith, G.L. & Moss, B. Proc. Natl. Acad. Sci. USA 79, 7415-7419 (1982).
20. Smith, G.L., Mackett, M. & Moss, B. Biotechnol. Genet. Eng. Rev. 2, 383-407 (1984).
21. Wiktor, T.J., MacFarlan, R.I., Reagan, K.J., Dietzschold, B., Curtis, P.J., Wunner, W.H., Kieny, M.P., Lathe, R., Lecocq, J.P., Mackett, M., Moss, B. & Koprowski, H. Proc. Natl. Acad. Sci. USA 81, 7194-7198 (1984).
22. Wiktor, T.J., Kieny, M.P. & Lathe, R. Appl. Virol. 2, in press (1986).
23. Bennink, J.R., Yewdell, J.W., Smith, G.L., Moller, C. & Moss, B. Nature 311, 578-579 (1984).
24. McMichael, A.J., Michie. C.A., Gotch, F.M., Smith, G.L. & Moss, B. J. Gen. Virol. 67, 719-726 (1986).
25. Hellstrom, K.E. & Hellstrom, I. Adv. Cancer Res. 12, 167-223 (1969).
26. Heberman, R.B. Adv. Cancer Res. 19, 207-263 (1974).
27. Treisman, R., Novak, U., Favaloro, J. & Kamen, R. Nature 292, 595-600 (1981).
28. Rassoulzadegan, M., Cowie, A., Carr, A., Glaichenhaus, N., Kamen, R. & Cuzin, F. Nature 300, 713-718 (1982).
29. Kieny, M.P., Lathe, R., Drillien, R., Spehner, D., Skory, S., Schmitt, D., Wiktor, T.J., Koprowski, H. & Lecocq, J.P. Nature 213, 163-166 (1984).
30. Takemoto, K.K., Malmgren, R.A. & Habel, K. Virology 28, 485-488 (1966).
31. Schaffhausen, B.S., Dorai, H., Arakere, G. & Benjamin, T.L. Molec. Cell. Biol. 2, 1187-1198 (1982).
32. Zhu, Z., Veldman, G.M., Cowie, A., Carr, A., Schaffhausen, B. & Kamen, R. J. Virol. 51, 170-180 (1984).
33. Dilworth, S.M., Hansson, H.A., Darnfors, C., Bjursell, G., Streuli, C.H. & Griffin, B.E. EMBO J. 5, 491-499 (1986).
34. Old, L.J., Clarke, D.A. & Benacerraf, B. Nature 184, 291-292 (1959).
35. Dalianis, T., Ramqvist, T. & Klein, G. Int. J. Cancer 34, 403-406 (1984).
36. Sharma, S., Rodgers, L., Brandsma, J., Gething, M.J. & Sambrook, J. EMBO J. 4, 1479-1589 (1985).
37. Koprowski, H., Herlyn, M., Steplewski, Z. & Sears, H.F. Science 212, 53-56 (1981).
38. Real, F.X., Mattes, M.J., Houghton, A.N., Oettgen, H.F., Lloyd, K.O. & Old, L.J. J. Exp. Med. 160, 1219-1233 (1984).
39. Ueda, R., Shiku, H., Pfreudschuh, M., Takahashi, L., Li, C., Whitmore, W.F., Oettgen, H.F. & Old. L.J. J. Exp. Med. 150, 564-572 (1979).
40. Drebin, J.A., Stern, D.F., Link, V.C., Weinberg, R.A. & Greene, M.I. Nature 312, 545-548 (1984).
41. Kieny, M.P., Lathe, R. & Lecocq, J.P. Gene 26, 91-99 (1983).
42. Lathe, R., Balland, A., Kohli, V. & Lecocq, J.P. Gene 20, 187-195 (1982).
43. Clertant, P., Gaudray, P., May, E. & Cuzin, F. J. Biol. Chem. 259, 15196-15203 (1984).

**Revendications**
**Revendications pour les Etats contractants suivants : AT, BE, CH, DE, FR, GB, IT, LI, LU, NL, SE**

1. Une composition pharmaceutique destinée au traitement préventif ou curatif d'une tumeur qui comprend à titre d'agent thérapeutique un vecteur viral recombinant choisi parmi les Poxvirus, les Adénovirus et les virus du groupe Herpès, comportant une séquence d'ADN hétérologue codant au moins pour la région essentielle d'une protéine spécifique de ladite tumeur appelée antigène T.

2. Une composition pharmaceutique selon la revendication 1, qui comprend à titre d'agent thérapeutique un vecteur viral recombinant choisi parmi les Poxvirus, les Adénovirus et les virus du groupe Herpès, comportant une séquence d'ADN hétérologue codant au moins pour la région essentielle d'une protéine spécifique de ladite tumeur, appelée antigène T.

3. Une composition pharmaceutique selon la revendication 1 ou 2, caractérisée en ce que le vecteur viral est le virus de la vaccine.

4. Une composition pharmaceutique selon l'une des revendications 1 à 3, caractérisée en ce que la séquence d'ADN codant pour un antigène T est une séquence d'ADN codant pour une protéine spécifique d'une tumeur humaine absente des tissus adultes normaux.

5. Une composition pharmaceutique selon l'une des revendications 1 à 3, caractérisé en ce que la séquence d'ADN codant pour un antigène T est une séquence d'ADN codant pour une protéine d'origine virale.

6. Une composition pharmaceutique selon la revendication 5, caractérisée en ce que la séquence d'ADN codant pour une protéine d'origine virale prodent d'un virus choisi parmi les papovavirus et les rétrovirus.

7. Une composition pharmaceutique selon l'une des revendications 1 à 6, caractérisée en ce qu'elle comporte un support pharmaceutiquement acceptable permettant son administration par injection à l'homme ou à l'animal.

**Revendications pour les Etats contractants suivants : ES, GR**

1. Utilisation d'un vecteur viral recombinant choisi parmi les Poxvirus, les Adénovirus et les virus du groupe Herpès, comportant une séquence d'ADN hétérologue codant au moins pour la région essentielle d'une protéine spécifique de ladite tumeur appelée antigène T, à titre d'agent thérapeutique pour la préparation d'une composition pharmaceutique destinée au traitement préventif ou curatif d'une tumeur.

2. Utilisation selon la revendication 1, dans laquelle le vecteur viral recombinant choisi parmi les Poxvirus, les Adénovirus et les virus du groupe Herpès, comporte une séquence d'ADN hétérologue codant au moins pour la région essentielle d'une protéine spécifique de ladite tumeur, appelée antigène T.

3. Utilisation selon la revendication 1 ou 2, caractérisée en ce que le vecteur viral est le virus de la vaccine.

4. Utilisation selon l'une des revendications 1 à 3, caractérisée en ce que la séquence d'ADN codant pour un antigène T est une séquence d'ADN codant pour une protéine spécifique d'une tumeur humaine absente des tissus adultes normaux.

5. Utilisation selon l'une des revendications 1 à 3, caractérisée en ce que la séquence d'ADN codant pour un antigène T est une séquence d'ADN codant pour une protéine d'origine virale.

6. Utilisation selon la revendication 5, caractérisée en ce que la séquence d'ADN codant pour une protéine d'origine virale provient d'un virus choisi parmi les papovavirus et les rétrovirus.

**7.** Utilisation selon l'une des revendications 1 à 6, caractérisée en ce qu'elle comporte un support pharmaceutiquement acceptable permettant son administration par injection à l'homme ou à l'animal.

**Claims**

**Claims for the following Contracting States : AT, BE, CH, DE, FR, GB, IT, LI, LU, NL, SE**

**1.** A pharmaceutical composition for the preventive or curative treatment of a tumor which comprises as therapeutical agent a viral vector chosen from amongst poxviruses, adenoviruses and viruses of the herpes group, which comprises a heterologous DNA sequence which codes at least for the essential region of a tumor-specific protein, called T antigen.

**2.** Composition as claimed in claim 1, in which the viral vector chosen amongst poxvirus, adenoviruses and viruses of herpes group comprises a heterologous DNA sequence coding at least for the essential region of a tumor-specific protein, called T antigen.

**3.** Composition as claimed in claims 1 or 2, which is a vaccine virus.

**4.** Composition as claimed in claims 1 and 2, wherein the DNA sequence coding for a T antigen is a DNA sequence coding for a protein which is specific for a human absent in normal adult tissues.

**5.** Composition as claimed in one of claims 1 to 3, wherein the DNA sequence coding for T antigen is a DNA sequence coding for a protein for viral origin.

**6.** Composition as claimed in claim 5, wherein the DNA sequence coding for the viral origin protein originates from a virus chosen from amongst papovaviruses and retroviruses.

**7.** Pharmaceutical composition as claimed in one of claims 1 to 6, which comprises a pharmaceutically acceptable vehicle which enables it to be administered by injection, to man or to animal.

**Claims for the following Contracting States : ES, GR**

**1.** Use of recombinant viral vector chosen from amongst poxviruses, adenoviruses and viruses of the herpes group, which comprises a heterologous DNA sequence which codes at least for the essential region of a tumor-specific protein, called T antigen as a therapeutical agent for preparing a pharmaceutical composition for the preventive or curative treatment of a tumor.

**2.** Use as claimed in claim 1, in which the viral vector chosen amongst poxvirus, adenoviruses and viruses of herpes group comprises a heterologous DNA sequence coding at least for the essential region of a tumor-specific protein, called T antigen.

**3.** Use as claimed in claims 1 or 2, which is a vaccine virus.

**4.** Use as claimed in claims 1 to 3, wherein the DNA sequence coding for a T antigen is a DNA sequence coding for a protein which is specific for a human tumor absent in normal adult tissues.

**5.** Use as claimed in one of claims 1 to 3, wherein the DNA sequence coding for T antigen is a DNA sequence coding for a protein from viral origin.

**6.** Use as claimed in claim 5, wherein the DNA sequence coding for viral origin protein originates from a virus chosen from amongst papovaviruses and retroviruses.

**7.** Use as claimed in one of claims 1 to 6, wherein the composition comprises a pharmaceutically acceptable vehicle which enables it to be administered by injection, to man or to animal.

**Patentansprüche**
**Patentansprüche für folgende Vertragsstaaten : AT, BE, CH, DE, FR, GB, IT, LI, LU, NL, SE**

1. Pharmazeutische Zusammensetzung für die präventive oder kurative Behandlung eines Tumors, die als therapeutisches Agens enthält einen rekombinanten Virus-Vektor, ausgewählt unter den Pockenviren, den Adenoviren und den Viren der Herpes-Gruppe, der eine heterologe DNA-Sequenz aufweist, die mindestens für den essentiellen Bereich eines spezifischen Proteins des genannten Tumors, als Antigen T bezeichnet, codiert.

2. Pharmazeutische Zusammensetzung nach Anspruch 1, die als therapeutisches Agens einen rekombinanten Virus-Vektor enthält, ausgewählt unter den Pockenviren, den Adenoviren und den Viren der Herpes-Gruppe, der eine heterologe DNA-Sequenz aufweist, die mindestens für den essentiellen Bereich eines spezifischen Proteins des genannten Tumors, als Antigen T bezeichnet, codiert.

3. Pharmazeutische Zusammensetzung nach Anspruch 1 oder 2, dadurch gekennzeichnet, daß es sich bei dem Virus-Vektor um das Vaccine-Virus handelt.

4. Pharmazeutische Zusammensetzung nach einem der Ansprüche 1 bis 3, dadurch gekennzeichnet, daß die DNA-Sequenz, die für ein Antigen T codiert, eine DNA-Sequenz ist, die für ein spezifisches Protein eines Human-Tumors codiert, das in den normalen Geweben von Erwachsenen fehlt.

5. Pharmazeutische Zusammensetzung nach einem der Ansprüche 1 bis 3, dadurch gekennzeichnet, daß die DNA-Sequenz, die für ein Antigen T codiert, eine DNA-Sequenz ist, die für ein Protein viralen Ursprungs codiert.

6. Pharmazeutische Zusammensetzung nach Anspruch 5, dadurch gekennzeichnet, daß die DNA-Sequenz, die für ein Protein viralen Ursprungs codiert, aus einem Virus stammt, das ausgewählt wird unter den Papova-Viren und den Retroviren.

7. Pharmazeutische Zusammensetzung nach einem der Ansprüche 1 bis 6, dadurch gekennzeichnet, daß sie einen pharmazeutisch akzeptablen Träger enthält, der ihre Verabreichung durch Injektion an Mensch oder Tier erlaubt.

**Patentansprüche für folgende Vertragsstaaten : ES, GR**

1. Verwendung eines rekombinanten Virus-Vektors, ausgewählt unter den Pockenviren, den Adenoviren und den Viren der Herpes-Gruppe, der eine heterologe DNA-Sequenz aufweist, die mindestens für den essentiellen Bereich eines spezifischen Proteins des genannten Tumors, als Antigen T bezeichnet, codiert, als therapeutisches Agens für die Herstellung einer pharmazeutischen Zusammensetzung zur präventiven oder heilenden Behandlung eines Tumors.

2. Verwendung nach Anspruch 1, in welcher der rekombinante Virus-Vektor, ausgewählt unter den Pockenviren, den Adenoviren und den Viren der Herpes-Gruppe, eine heterologe DNA-Sequenz aufweist, die mindestens für den essentiellen Bereich eines spezifischen Proteins des genannten Tumors, als Antigen T bezeichnet, codiert.

3. Verwendung nach Anspruch 1 oder 2, dadurch gekennzeichnet, daß es sich bei dem Virus-Vektor um das Vaccine-Virus handelt.

4. Verwendung nach einem der Ansprüche 1 bis 3, dadurch gekennzeichnet, daß die DNA-Sequenz, die für ein Antigen T codiert, eine DNA-Sequenz ist, die für ein spezifisches Protein eines Human-Tumors codiert, das in normalen Geweben von Erwachsenen fehlt.

5. Verwendung nach einem der Ansprüche 1 bis 3, dadurch gekennzeichnet, daß die DNA-Sequenz, die für ein Antigen T codiert, eine DNA-Sequenz ist, die für ein Protein viralen Ursprungs codiert.

6. Verwendung nach Anspruch 5, dadurch gekennzeichnet, daß die DNA-Sequenz, die für ein Protein viralen Ursprungs codiert, aus einem Virus stammt, der ausgewählt wird unter den Papova-Viren und den Retroviren.

7. Verwendung nach einem der Ansprüche 1 bis 6, dadurch gekennzeichnet, daß sie einen pharmazeutisch akzeptablen Träger umfaßt, der die Verabreichung durch Injektion an Mensch oder Tier erlaubt.

FIG-1

EP 0 259 212 B1

FIG-2

FIG.3